(19) Europäisches Patentamt
European Patent Office
Office européen des brevets

(11) Numéro de publication: **0 179 689**
**B1**

(12) **FASCICULE DE BREVET EUROPÉEN**

(45) Date de publication du fascicule du brevet: **12.10.88**

(51) Int. Cl.⁴: **C 07 D 307/24**, C 07 D 307/84, C 07 D 333/38, C 07 C 102/06, C 07 C 103/737

(21) Numéro de dépôt: **85401845.4**

(22) Date de dépôt: **23.09.85**

(54) **Procédé de préparation de diamines substituées.**

(30) Priorité: **08.10.84 FR 8415405**

(43) Date de publication de la demande: **30.04.86 Bulletin 86/18**

(45) Mention de la délivrance du brevet: **12.10.88 Bulletin 88/41**

(84) Etats contractants désignés: **AT BE CH DE FR GB IT LI LU NL SE**

(56) Documents cité: **WO-A-79/00628** **FR-A-2 466 462**

(73) Titulaire: **SYNTHELABO, 58 rue de la Glacière, F-75013 Paris (FR)**

(72) Inventeur: **Rossey, Guy, 8, Square Lebrun Voisins-le- Bretonneux, F-78180 Montigny- le- Bretonneux (FR)**
Inventeur: **Michel, Jean- Claude, 4, Square Michelet, F-95100 Argenteuil (FR)**

(74) Mandataire: **Thouret- Lemaitre, Elisabeth, Service Brevets - SYNTHELABO 58, rue de la Glacière, F-75621 Paris Cédex 13 (FR)**

**EP 0 179 689 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne un nouveau procédé de préparation de diamines substituées.

Les diamines substituées répondent à la formule suivante

$R^2HN - C_nH_{2n} - NHCOR^1$ (I)

dans laquelle

n va de 2 à 6,

$R^1$ représente un radical $(C_{3-6})$cycloalkyle, tétrahydrofuryle, benzofuryle, benzotétrahydrofuryle ou thiényle,

$R^2$ représente un radical $(C_{1-4})$alkyle.

Les diamines substituées obtenues selon le procédé de l'invention sont des produits intermédiaires utilisés pour la préparation d'alkylènediamines décrites par la demanderesse dans son brevet français n°78 03 175 et les certificats d'addition n°78 36 819 et n°79 24 373.

Un procédé de préparation d'amides aromatiques antioxydants est décrit dans la demande de brevet n° WO 79/00628, à partir d'une diamine aromatique et d'un ester, en présence d'une base et d'un alcool, dans un solvant.

Le procédé de l'invention est décrit dans le schéma donné ci-après

$$R^2HN - C_nH_{2n} - NH_2 + R^3OOC-R^1 \quad (R^3 = CH_3, C_2H_5)$$

(II) (III)

$$\downarrow$$

$$R^2HN - C_nH_{2n} - NHCOR^1 \quad (I)$$

Il consiste à faire réagir une ω-alkylamino-alkylène-amine de formule (II) avec un ester (III), en l'absence de solvants, à une température de 20 à 130°C.

Les amines (II) sont disponibles dans le commerce. Les esters (III) sont préparés à partir de produits disponibles dans le commerce et sont décrits dans la littérature.

L'exemple suivant illustre l'invention. Les analyses et les spectres IR et RMN confirment la structure des composés.

Exemple N- [(méthylamino)-3 propyl] -tetrahydrofuranne-carboxamide-2.

[$R^2 = CH_3$ ; n = 3 ; $R^1$ = 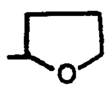 ]

1. Le tétrahydrofurannecarboxylate-2 de méthyle, ester de formule (III), est décrit par Wilson, J. Chem. Soc. 58, 59 (1945).

2.N- [(méthylamino)-3 propyl]-tétrahydrofurannecarboxa-mide-2.

Dans un réacteur de 25 ml, on agite à 30°C un mélange de 13 g de tétrahydrofurannecarboxylate-2 de méthyle (obtenu sous 1) et de 9 g de N-méthyl-propanediamine pendant 6 h. On maintient l'agitation à la température ambiante pendant 16 h.

On distille sous pression réduite le mélange homogène incolore.

On obtient une huile incolore qui bout à 115-117°C.

Le procédé de l'invention permet d'obtenir les composés (I) avec un excellent rendement allant de 70 à 100 % par rapport aux composés de départ et, en conséquence, d'obtenir les alkylènediamines décrites par la demanderesse avec de très bons rendements, par réaction entre les composés (I) obtenus selon le procédé de l'invention et la chloro-2 amino-4 diméthoxy-6, 7 quinazoline.

**Revendication**

Procédé de préparation de diamines substituées répondant à la formule (I)

$R^2HN - C_nH_{2n}- NHCOR^1$ (I)

dans laquelle n va de 2 à 6,
$R^1$ représente un radical $(C_{3-6})$cycloalkyle, tétrahydrofuryle, benzofuryle, benzotétrahydrofuryle ou thiényle.
$R^2$ représente un radical $(C_{1-4})$alkyle, procédé caractérisé en ce que l'on fait réagir un composé (II)

$R^2HN - C_nH_{2n} - NH_2$

avec un composé $R^3OOC-R^1$ (III), ($R_3 = CH_3$ ou $C_2H_5$), à une température de 20 à 130°C.

**Patentanspruch**

Verfahren zur Herstellung von substituierten Diaminen der Formel (I)

$R^2HN - C_nH_{2n} - NHCOR^1$ (I)

worin n 2 bis 6 bedeutet, $R^1$einen $(C_{3-6})$-Cycloalkyl-, Tetrahydrofuryl-, Benzofuryl-, Benzotetrahydrofuryl- oder Thienylrest und $R^2$ einen $(C_{1-4})$-Alkylrest darstellt,
dadurch gekennzeichnet,
daß man eine Verbindung (II)

$R^2HN - C_nH_{2n} - NH_2$

mit einer Verbindung $R^3OOC-R^1$ (III), ($R^3 = CH_3$ oder $C_2H_5$) bei einer Temperatur von 20 bis 130°C reagieren läßt.

**Claim**

Process for the preparation of substituted diamines corrresponding to the formula (I)

$R^2HN-C_nH_{2n}-NHCOR^1$ (I)

in which
n ranges from 2 to 6,
$R^1$ represents a $C_{3-6}$-cycloalkyl, tetrahydrofuryl, benzofuryl, benzotetrahydrofuryl or thienyl radical and
$R^2$ represents a $C_{1-4}$-alkyl radical, characterised in that a compound (II)

$R^2HN-C_nH_{2n}-NH_2$

is reacted with a compound $R^3OOC-R^1$ (III), ($R^3 = CH_3$ or $C_2H_5$), at a temperature of 20 to 130°C.